# EUROPEAN PATENT SPECIFICATION

(11) **EP 2 571 462 B1**
(45) Date of publication and mention of the grant of the patent: **31.12.2014**
(21) Application number: 11724575.3
(22) Date of filing: 19.05.2011
(51) Int. Cl.: A61F 5/01, A61F 5/02

(54) **HIP SUPPORTING DEVICE**
HÜFTSTÜTZENDE VORRICHTUNG
DISPOSITIF DE SUPPORT DE HANCHE

(30) Priority: 19.05.2010 US 34607010 P; 19.05.2010 EP 10163300
(43) Date of publication of application: 27.03.2013
(73) Proprietor: Technical University of Denmark, 2800 Lyngby (DK)
(72) Inventor: BRØNDSTED, Povl, DK-4600 Køge (DK); KOT, Katrine Kirkeskov, DK-4130 Viby Sj. (DK)
(74) Representative: Høiberg, Susanne
(86) International application number: PCT/EP2011/058146
(87) International publication number: WO 2011/144698

(56) References cited:
- DE-A1- 3 904 491
- US-A- 1 722 192
- US-A- 5 709 648

## Description

The present invention relates to a device for limiting movements in one or more anatomical joints, such as a device for limiting movement in the human hip joint after hip replacement surgery. Document WO-A-2009/017859 is regarded as the closest prior art.

### Background of invention

As the population ages, it has created a greater demand for orthotic management of any hip joint that has been compromised. Following a hip joint replacement, it is important to provide proper post-operative treatment of the patient after arthoplatyies.

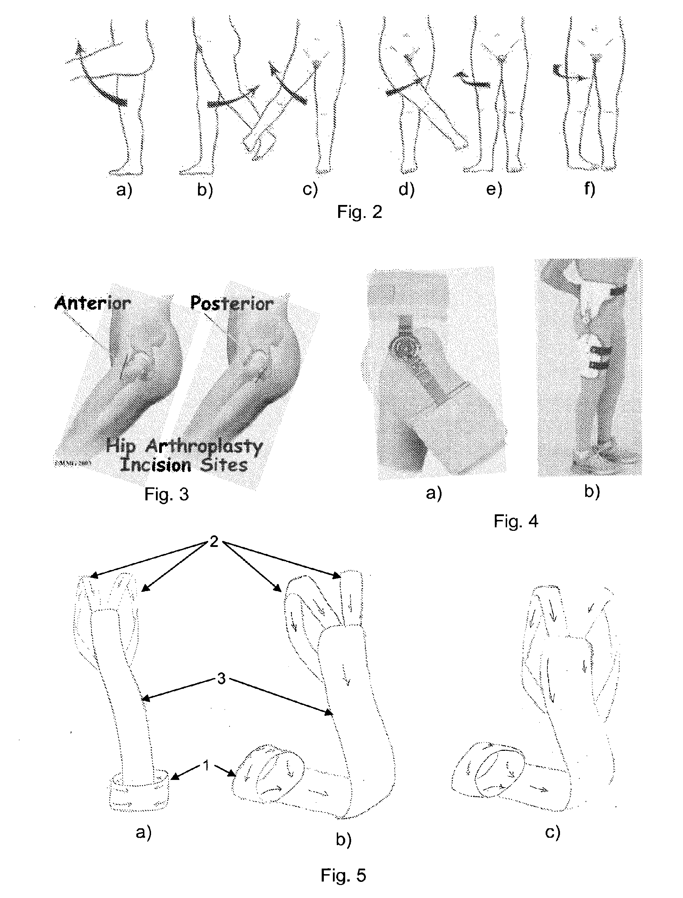

Orthotic management of hip joint compromise has been a challenge for orthopaedics, orthotists and therapists when dealing with patients whose hip joints and their associated soft tissues, joint integrity, alignment, and bone and capsular components are compromised. A hip is a multidirectional joint capable of flexion, extension, internal and external rotation, adduction, and abduction (see fig. 2). In addition to its mobility, the hip joint must absorb the force of full weight-bearing and provide stability to the pelvis both for standing and for single support during gait. Additionally, during walking, while one hip is stabilized, the opposite leg must have the strength, range of motion, and structural integrity to advance.

One or more muscle groups of the hip joint are compromised when surgical procedures are performed at the hip joint, especially during a hip replacement surgery. A significant problem that occurs when a hip joint has been compromised is dislocation of the hip joint: The femoral head can be driven out of the acetabulum. The hip is most susceptible to posterior dislocation when it is flexed past 90 degrees, internally rotated and adducted (see fig. 2). Examples of this action occur in every day living, such as sitting on a low chair and leaning forward while putting weight on the affected hip joint and internally rotating when coming to a standing position. Thus, common activities of daily living, specifically excessive hip flexion with loaded extremity and internal rotation on the affected side, can cause dislocation. Anterior dislocation also occurs when a hip is externally rotated, abducted, and flexed and if, for example, a knee is subject to a force, such as accidentally hitting an object. The neck of the femur or the greater trochanter levers the femur out of the acetabulum. To avoid these problems, a hip supporting device must be able to effectively control the limits of extension and rotation in a patient who has experienced an anterior dislocation.

### Summary of invention

An example of a modular, adjustable prophylactic hip orthosis can be found in US 7,048,707. The orthosis provides a substantially rigid pelvic or hip engaging unit that is formed to conform to the contours of the human hip. As disclosed in WO 2009/017949 this orthosis has been further developed with an adjustable extension posterior spinal orthosis with a pair of adjustable shoulder straps connected to the top of the extension orthosis and fastened at the bottom to the rear connector plate, thereby providing an improved control of both extension, flexion and abduction and adduction of the hip joint.

However, this does not remove the discomfort experienced by the user wearing a rigid hip engaging unit. One object of the invention is therefore to provide a more comfortable hip supporting unit to encourage maximum prolonged usage.

This is achieved by a device for limiting movement in the human hip joint comprising the features of claim 1.

In the preferred embodiment of the invention the first member is a collar, preferably adapted to conform to the thigh of a user. Further, the second member is preferably one or more shoulder straps. The third member is preferably at least one band connecting the first member (e.g. leg collar(s)) and the second member (e.g. shoulder strap(s)).

The invention can be used by a method for limiting one or more movements in the hip joint of a user by anchoring the tensile load from said one or more hip joint movements in one or both of the shoulders. The tensile load is preferably distributed to the shoulders by means of a soft and flexible element. This element may be substantially inelastic. The element is preferably comfortable to wear for the user. Thus, the tensile load may be anchored in one or both shoulders by means of e.g. a band fixed to one or both of said shoulders and one or both legs of the user.

As such the present invention thus may provide a comfortable hip supporting device without components actually located on the hip. In particular the user avoids wearing a rigid shell on the hip where specific hip joint movements are limited partly by the rigidity of the rigid shell and partly by rotary mechanical joint (hinge) between the rigid shell parts. In the present invention one or more specific leg movements are limited by comfortable soft and/or flexible band(s) and straps that limit these movements and distribute the tensile pull of these "limitations" to the shoulder part of the user.

### Definitions (Wikipedia)

**The hip joint** is a synovial ball and socket joint that consists of the articulation of the spherical head of the femur with the cup-like shape of the acetabulum. An acetabular labrum attaches to the bony rim of the acetabulum and cups around the head of the femur to hold it firmly in place. Various ligaments add strength to the articulation of the hip joint and a large number of muscles act on the hip joint. The gluteus medius is primarily associated with abduction. Anterior fibers assist with flexion and internal rotation. Posterior fibers assist with extension and external rotation. These muscle groups stabilize the pelvis during a single leg support.

**Hip replacement** is a surgical procedure in which the hip joint is replaced by a prosthetic implant. Replacing the hip joint consists of replacing both the acetabulum and the femoral head. Such joint replacement orthopaedic surgery generally is conducted to relieve arthritis pain or fix severe physical joint damage as part of hip fracture treatment. Hip replacement is currently the most successful and reliable orthopaedic operation with approx. 97% of patients reporting improved outcome.

### Detailed description of the invention

In the preferred embodiment of the invention the shoulder engaging member (i.e. the second member) comprises one or more shoulder straps. Further the shoulder strap(s) may comprise one or more closing mechanisms, thereby easing mounting and dismounting of the device. Further, means for adjusting the length of the shoulder strap(s), such as one or more buckles, may be provided, thereby providing the option of adapting the device to the specific physical dimensions of a user. Individual adaptation may further be provided by at least partly elastic shoulder straps.

The third member is provided to distribute the tensile pull provided by hip joint movements to one or both of the shoulders. As previously indicated this may be provided by a band or equivalently a webbing or strop or belt. The band is preferably adapted to extend along at least a part of the back or the chest of a user. Further, the band is preferably adapted to extend further along at least one buttock or groin of a user. The location of the band (back - buttock or chest - groin) depends on which movements the device is supposed to limit. To limit flexion in the hip joint the band is preferably adapted to extend along the back, at least partly along the spinal column, across the hip and the buttock and further on to the back of the thigh where the band connects to the leg conforming member. Accordingly: to limit extension in the hip joint the band is preferably adapted to extend the chest across the hip and the groin and further on to the front of the thigh where the band connects to the leg conforming member. The advantage of using a band is that a band can be soft and flexible and thereby comfortable to wear for the user. Combined with these comfortable features the band may be provided with the necessary resilience towards being extended in the longitudinal direction, such that when the band is pulled it will resist and distribute the tensional load to the second member and the shoulder part and thereby prevent a user from e.g. flexion in the hip joint.

In the preferred embodiment of the invention the third member is substantially inelastic. But the third member may however in other embodiments be provided with a small elasticity, like the elasticity seen in dense textile like twill fabric.

In a further embodiment of the invention the second member is provided with visible distinguishable markings.

Thus, in order to limit flexion or extension it suffers to provide some sort of connection between the leg conforming member and the shoulder engaging member. However, further limitations to hip joint movements may be provided if the band is substantially wide, preferably both substantially flat and wide and elongated. A wide band may further limit abduction, adduction and rotation of the hip joint. However, if the band is too wide it may be uncomfortable to wear and there may further be bacteriological issues if the band is inconvenient during toilet visits. Thus, in one embodiment of the invention the third member is provided with a narrow end adapted to conform to a buttock and a leg of a user. I.e. the third member thereby has a wider part adapted to extend along the back (or chest) of the user and a less wide part adapted to conform to the buttock (or groin) and leg area. The maximum width of the band may be between 0.2 and 1.5 times the maximum diameter of the thigh of the user, such as between 0.3 and 1.4 times the maximum diameter of the thigh of the user, such as between 0.4 and 1.3 times the maximum diameter of the thigh of the user, such as between 0.5 and 1.2 times the maximum diameter of the thigh of the user, such as between 0.6 and 1.2 times the maximum diameter of the thigh of the user, such as between 0.7 and 1.1 times the maximum diameter of the thigh of the user, such as between 0.8 and 1.1 times the maximum diameter of the thigh of the user, such as between 0.9 and 1.1 times the maximum diameter of the thigh of the user.

In one embodiment of the invention the width of the third member is at least 5 cm, such as at least 6 cm, such as at least 7 cm, such as at least 8 cm, such as at least 9 cm, such as at least 10 cm, such as at least 11 cm, such as at least 12 cm, such as at least 13 cm, such as at least 14 cm, such as at least 15 cm, such as at least 16 cm, such as at least 17 cm, such as at least 18 cm, such as at least 19 cm, such as at least 20 cm.

In one embodiment of the invention the maximum width of the third member is between 5 and 30 cm, such as between 7 and 24 cm, such as between 10 and 22 cm, such as between 11 and 21 cm, such as between 12 and 20 cm, such as between 13 and 19 cm, such as between 14 and 18 cm, such as between 15 and 17 cm, such as between 13 and 18 cm, such as between 14 and 19 cm, such as between 15 and 20 cm, such as between 16 and 21 cm, such as between 17 and 22 cm, such as between 18 and 23 cm, such as between 19 and 24 cm, such as between 20 and 25 cm, such as between 21 and 26 cm, such as between 22 and 27 cm, such as between 23 and 28 cm.

In one embodiment of the invention the width of the shoulder straps is between 2 and 12 cm, such as between 3 and 11 cm, such as between 4 and 10 cm, such as between 5 and 9 cm, such as between 6 and 8 cm, such as approx. 7 cm.

In one embodiment of the invention the band is adapted to be substantially taut when the device is worn by a user. This may help to ensure that any hip joint movement is limited, because the taut band applies tension to the collar and the shoulder straps, i.e. the movement of the leg is limited because the taut band distributes the tensional pull to one or more of the shoulders.

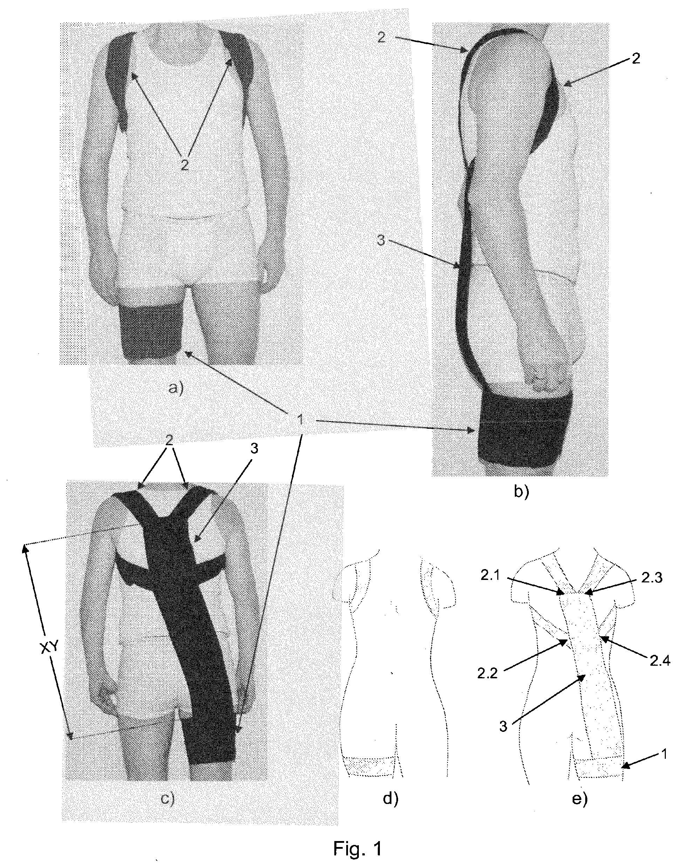

The length of the third member, i.e. the connecting member, i.e. preferably a band, can be determined by the height of the user. In the ideal configuration of the device according to the invention the length of the third member substantially corresponds to the distance between a point between the shoulder blades to the midpoint of the thigh of the user. This length is indicated as XY in fig. 1c, i.e. the length from the top of the band where the shoulder straps are attached to the collar. For a user with a height around 180 cm the appropriate length of the third member is approx. 70 cm. There may be large physiological differences between different users, also between different users of equal height, but as a rule of thumb the length of the third member may approx. correspond to around 40% of the height of the user, such as between 35% and 45% of the height of the user.

In a further aspect of the invention the length of the band is between 40 and 100 cm, such as between 50 and 90 cm, such as between 55 and 85 cm, such as between 60 and 80 cm, such as between 63 and 77 cm, such as between 65 and 75 cm, such as between 67 and 73 cm, such as between 69 and 71 cm.

As hip joint movements originate from movements of the femur it seems reasonable to anchor the device to the thigh. Thus, in the preferred embodiment of the invention the leg conforming member is a collar, preferably this collar is adapted to conform around a thigh, preferably adapted to fit tightly around the lower part of the thigh just above the knee. The leg conforming member may be attached to the thigh at any position, however attachment close to the crotch may be discomforting to a user. The width of the collar is preferably approx. one third of the length of the femur (thigh bone) of the user, such as between 0.3 and 0.4 times the length of the femur of the user, such as between 0.25 and 0.5 times the length of the femur of the user, such as between 0.2 and 0.6 times the length of the femur of the user.

A thigh will typically be thinnest at the knee with a cone like shape from the knee and up. By providing a collar around the lower part of the thigh the natural cone like shape of the thigh will prevent the collar from sliding up and thereby maintain the tension in the device. The device may be more comfortable to the user if the shape of the collar is substantially conical.

The collar may be a closed collar and mounting of the device may be provided by sticking the foot trough the collar and sliding it up to the thigh. However, it may be easier for the user if the collar is provided with at least one closing mechanism, such as fabric hook-and-loop fasteners, such as Velcro, and thereby providing for an individual adaptation to the diameter of the thigh. The collar may be a band extending around the thigh. The width of the collar (equivalent to the height of the collar when worn by a user) is a balancing between comfort for the user and stability of the attachment. A wide collar covering the whole thigh may provide a very firm attachment, however less comfort for the user. A narrow collar may provide more comfort for user but an unstable attachment to the thigh. Thus, in one aspect of the invention the width of a collar may be between 5 and 26 cm, such as between 7 and 24 cm, such as between 10 and 22 cm, such as between 11 and 21 cm, such as between 12 and 20 cm, such as between 13 and 19 cm, such as between 14 and 18 cm, such as between 15 and 17 cm.

Even though the third member is preferably substantially wide and thereby also adapted to limit abduction, adduction, and lateral and medial rotation in the hip joint it may be necessary to provide additional limitations against one or more of these hip joint movements. Thus, in a further embodiment of the invention the device comprises a fourth member attached to the first member and the upper part of the third member. The fourth member is preferably substantially soft and/or flexible and adapted to limit one or more specific movements of said leg by anchoring the tensile load of said movement(s) in the shoulder(s). Further, the fourth member is preferably adapted to extend from the back of a user to the front side of the thigh of the user. Preferably the fourth member is adapted to limit abduction or adduction in one side of the hip joint. The fourth member may be a strap. Preferably the fourth member is substantially inelastic. Thus, the fourth member may be a strap that is attached to the upper part of the third member (preferably where the second member is attached) and that is adapted to extend down, preferably substantially diagonal, along the back of a user and round the outside of the buttock and the thigh down to the lower front part of the thigh where the fourth member is attached to the first member, which may be a collar. If a user then tries to adduct, i.e. cross the legs, the fourth member will help to limit this movement and the tensional load is distributed to the shoulder part via the third member.

The device may be regarded as an orthosis or more specifically a hip orthosis. However, in the device may also be regarded as a bandage.

In the preferred embodiment of the invention the device is adapted to be worn on the skin of the user. However, in some cases it may se suitable to wear the device over some kind of fabric, such as the clothes. Thus, the device is preferably at least partly provided in a biocompatible material, such as cotton. The device may as such be washable.

Selecting the right material for the device is a compromise between different parameters such as comfort, strength, elasticity, biocompatibility and cost. Textile as the primary material has proven to provide such a good compromise. Thus, in one embodiment of the invention the device, or at least the first member and/or the third member, is primarily manufactured in textile selected from the group of fabric, cloth, woven fabric, twill, denim, chino, drill, gabardine, tweed, canvas and serge. First of all users are accustomed to wearing textile, which is important because the device may be worn for 24 hours a day for 3 months. Secondly textile may provide a sufficient strength in order to efficiently oppose the substantial muscle power that may be exerted by users e.g. during flexion or extension in the hip joint. Thirdly textile may be provided as marginally elastic. Too much elasticity may pose a risk when e.g. flexion over 90 degrees must be avoided. And typically textile is biocompatible, washable and cost efficient.

In the preferred aspect of the invention the device limits flexion and/or extension of one or both sides of the hip joint of a user, i.e. when the device is worn by a user. Further, the device may limit abduction and/or adduction of one or both sides of the hip joint of a user. Even further the device may limit lateral rotation and/or medial rotation of one or both sides of the hip joint of a user.

### Drawings

The invention will now be described in more detail with reference to the drawings in which
- Figs. 1 a-c: show photos of the first embodiment of the invention,
- Figs. 1 d-e: are illustrations of the first embodiment,
- Figs. 2a-f: are illustrations of the hip joint movements,
- Fig. 3: shows hip arthroplasty incision sites,
- Figs. 4a-b: show prior art hip orthoses,
- Figs. 5a-c: are illustrations of the tensional forces in the first embodiment of the invention,
- Figs. 6a-c: show photos of a second embodiment of the invention,
- Figs. 6d-e: are illustrations of the second embodiment,
- Figs. 7a-b: show illustrations of a third embodiment of the invention,
- Figs. 8a-b: show illustrations of a fourth embodiment of the invention,
- Figs. 9a-b: show illustrations of a fifth embodiment of the invention,
- Figs. 10a-b: show examples of where shoulder straps may attach to a band,
- Fig. 11: shows an unfolded illustration of the first embodiment,
- Figs. 12a-d: show photos of a sixth embodiment of the invention,
- Figs. 13a-c: show close-up photos of the sixth embodiment,
- Fig. 14: shows a photo of a shoulder strap support across the chest, and
- Fig. 15: shows a photo of markings on a shoulder strap.

### Detailed description of the drawings

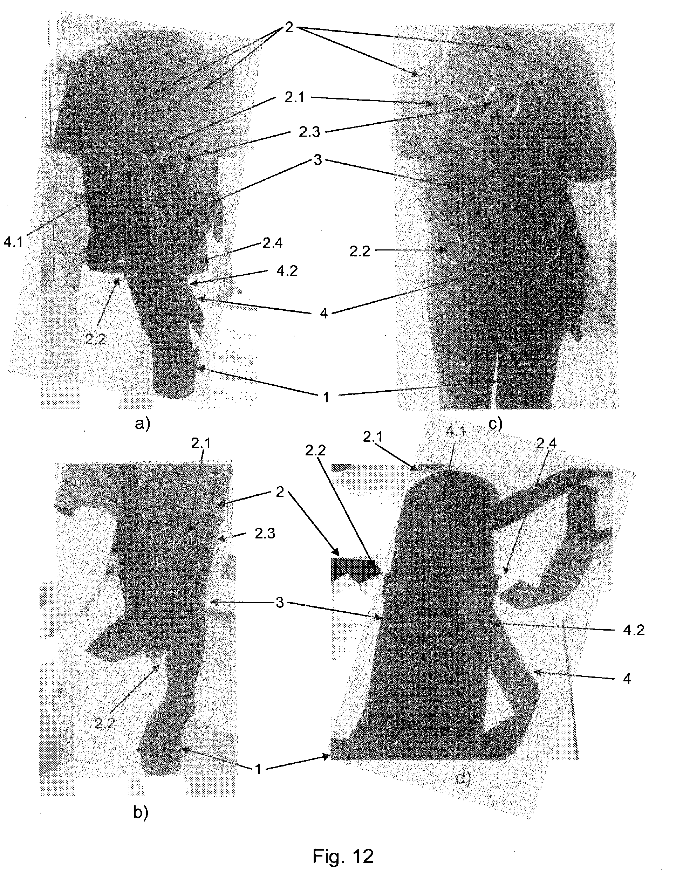

A first embodiment of the invention is shown in fig. 1 and a comparable sixth embodiment is shown in fig. 12. Figs. 1a)-c) show photos from the front, side and back, respectively, and fig. 12 show photos substantially from the back, of users wearing the device. In the shown cases the users are wearing the device to limit the movements in the right side of the hip joint. A collar 1 is wrapped around the lower part of the right thigh and shoulder straps 2 extend around both shoulders. A wide band 3 connects the collar 1 with the shoulder straps 2. The shoulder straps 2 are attached to the band 3 near the shoulder blades and the band 3 extends along the spinal column with a small twist towards the right side, passes the hip and around the right buttock of the user. If the user flexes his right hip joint, e.g. by lifting the right leg, the device will limit this movement. Because the collar 1 (attached to the thigh) will pull the band 3 and thereby the shoulder straps 2 and thereby distribute at least a part of the tensile load exerted by the user lifting his leg to the shoulders of said user. By providing a suitable length, tension and strength of the band 3 the user can be limited to e.g. a 90 degree flexion of the right hip joint.

In this case the user has probably received a posterior hip incision in the right side of the hip that necessitates limitation of flexion in the right hip joint. With a posterior incision there is no need to limit extension of the hip joint and the device as shown in fig. 1 does not limit extension of the hip joint. However, if a limitation in extension is needed the device may be "reversed", i.e. by having the band across the chest. If limitation in both flexion and extension is needed there may be bands across both back and chest.

From fig. 1 it can be observed that the height of the collar in this embodiment takes up between one third and one half of the length of the thigh. This is to ensure a firm grip of the collar and good comfort for the user. In this example the width of the band is substantially equal to the height of the collar. When the band is taut the tensional force is distributed all across the width. A wide band therefore helps to distribute the tensional load across a large area. A narrow band could be uncomfortable for the user, e.g. across the buttock. The width of the band also helps to limit abduction, adduction, and rotation of the hip joint. A rotation of a leg corresponds to a lateral or a medial rotation in the hip joint. If a user wearing the device rotates the leg it corresponds to a distortion of the band like a spiral. A wide band which is taut will provide more resistance when distorted than a narrow taut band. Thus, the device according to the preferred embodiment of the invention will limit rotation in the hip joint. The same applies to abduction and adduction in the hip joint: A wide band will limit these movements. From fig. 1 it can be seen that the width of the band approx. corresponds to the diameter of the thigh of the user.

From figs. 1 a-c it is seen that the device is worn partly on the skin and partly over the underwear.

The first embodiment is also illustrated in figs. 1d (front) and 1 e (back). From Fig. 1e it seen that the shoulder straps 2 are attached to the band 3 in four attachment sites: two attachment sites for the left shoulder strap 2.1, 2.2 and two attachment sites for the left shoulder strap 2.3, 2.4. Equivalent to two upper attachment sites 2.1, 2.3 and two lower attachment sites 2.2, 2.4. These four attachment sites are also visible in fig. 1c. These attachment sites are also marked in fig. 12.

Six different movements of the hip joint are illustrated in fig. 2 with flexion in fig. 2a, extension in fig. 2b, abduction in fig. 2c, adduction in fig. 2d, lateral rotation in fig. 2e and medial rotation in fig. 2f.

Fig. 3 illustrates two possible incision sites in hip replacement surgery. To the left is shown an anterior incision site and to the right is shown a posterior incision site. The posterior incision is the most common. Approx. nine out of ten hip replacement surgical operations is performed with a posterior incision because this procedure provides the best result in terms of patient mobility after surgery. Hip replacement surgery typically requires limitations in hip joint movement for approx. 3 months after surgery, often 24 hours per day. Typical required restrictions after hip replacement surgery can be to avoid more than 90 degrees flexion, adduction and medial rotation. In particular any combination of these movements is critical to avoid.

Fig. 4 show hip orthoses known in the art from Donjoy (fig. 4a) and Orthomerica (fig. 4b). These orthoses are based on rigid hip and leg shells connected by metal rods engaged by a hinge that limits flexion and/or extension of the hip joint. Needles to say these known devices are uncomfortable to wear for the patients, especially when worn for 24 hours per day. A further problem with these rigid shell devices is that the patient movement may be limited too much. An important part of rehabilitation after hip replacement surgery is training of the surrounding muscles, e.g. provided by abduction and extension of the hip joint, which is allowed after posterior incision surgery. However, the devices shown in fig. 4 do not allow abduction and extension of the hip joint.

The arrows in fig. 5 illustrate the tensional forces of the first and sixth embodiments of the invention in different positions of the user (the user is not shown). Fig. 5a shows the device when the user is standing straight, in fig. 5b the user is sitting and in fig. 5c the user is turning the torso while sitting.

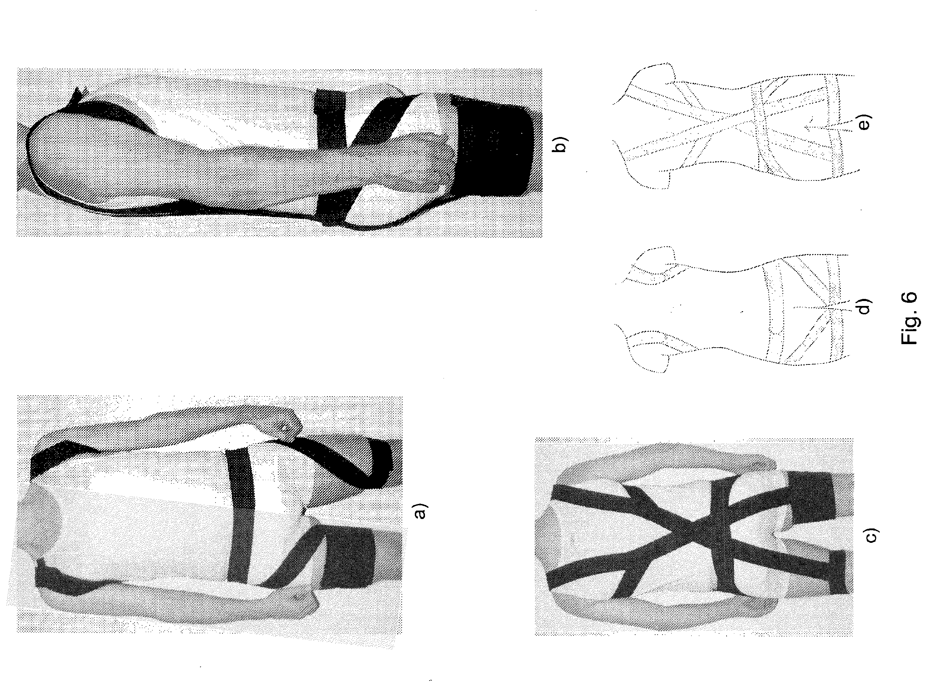

Fig. 6 shows a second embodiment of the invention with front, side and back photos in figs. 6a, 6b and 6c, respectively. In this embodiment two leg collars are provided to distribute the tensional forces. In this case the right hip joint has undergone surgery, and to further stabilize the hip a collar for the left leg is also provided. Compared to the first embodiment shown in fig. 1 two bands are provided extending from the back of each side of the hip, around each thigh and attached to each leg collar at the inner thigh. These bands are provided to further limit adduction and abduction. A band around the hip is further provided as a belt. Compared to the first embodiment this second embodiment may provide increased stabilization of the hip joint, however compromising the comfort and usability for the user, because mobility can be too much decreased. In particular it can be annoying to limit movement of the healthy side of the hip joint.

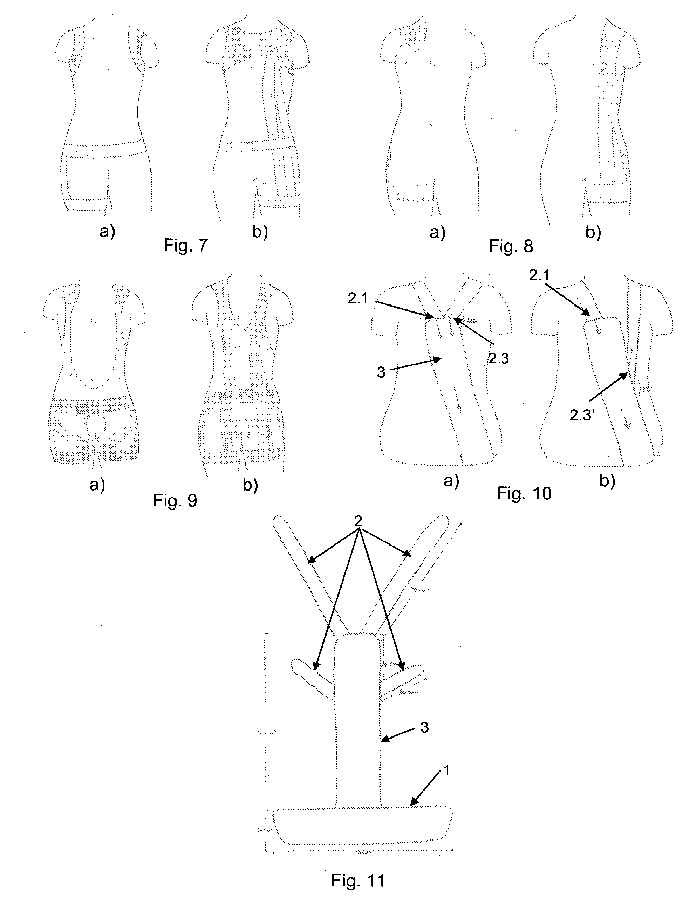

Fig. 7 illustrates a third embodiment of the invention with a front view in fig. 7a and a back view in fig. 7b. The shoulder part is like a tank top that is provided to distribute the tensional load all over the shoulders. Two bands are attached to the same point on the shoulder part. The first band extends along the back and over the buttock down to a leg collar while the second band extends along the side of the user to the leg collar. The first band limits flexion on the right hip joint whereas the second band limits adduction of the right hip joint. A band is located like a belt around the hip/waist to keep the two vertical bands in place. The advantage of this third embodiment is that the tensional load is more widely distributed across the shoulders due to the tank top shaped shoulder part. However, this shoulder part may also be less comfortable to a user and may also reduce the user's mobility.

Fig. 8 illustrates a fourth embodiment of the invention with a front view in fig. 8a and a back view in fig. 8b. The tensional load is anchored around only one shoulder. A band along the side of the user splits into two bands that extend over the buttock and over the side of the hip, respectively, before attachment to a leg collar. The advantage of this solution is that the healthy side of the body is completely unrestricted providing a good mobility and good comfort. However, with the tensional load on only one shoulder the user will experience a constant unsymmetrical pull in the body which may be uncomfortable for a longer period of use.

Fig. 9 illustrates a fifth embodiment of the invention with a front view in fig. 9a and a back view in fig. 9b. This embodiment resembles the second embodiment with collars on both legs and bands that extends cross the thighs. Compared to the second embodiment this fifth embodiment is integrated in a garment that resembles cycling bib shorts. This solution may provide enhanced support to the various joints and may help to keep the various device components in place. However, it may be uncomfortable to wear a suit like this for an extended period.

Fig. 10 illustrates possible variations for attaching shoulder straps to a band. In the first embodiment as illustrated in fig. 1 the tensional pull in the shoulders is not equally distributed between the left and right shoulder. Due to the substantially diagonal band as illustrated in fig. 1 the tensional pull from e.g. lifting the leg on the supported side of the hip will be felt in the opposite shoulder, i.e. if the user in fig. 1 lifts his right leg the tensional pull will be strongest in the left shoulder. This unequally distributed tensional pull may be equalled by changing the upper attachment site of the shoulder strap in the same side as the supported hip. Fig. 10a illustrates the shoulder strap attachments equivalent to fig. 1e, i.e. with the upper attachment sites 2.1, 2.3 located next to each at the end of the band 3, i.e. approx. in between the shoulder blades. Please note that the lower attachment sites 2.2, 2.4 are not illustrated in fig. 10. The tensional pull in the shoulders may be more equally distributed if the tensional pull in the same side as the supported hip was a more direct pull, i.e. if the angle between the band and the shoulder strap was close to 180 degrees, or at least closer to 180 degrees than illustrated in fig. 1e and fig. 10a. This may be provided by changing the upper attachment site of the shoulder strap and move it to a position further down the band, whereby the angle between the shoulder strap and the band increases. An example is illustrated in fig. 10b wherein the upper shoulder strap attachment site in the same side as the supported hip has been moved to a position 2.3', i.e. approx. near the waistline of the user. This changes the distribution of the tensional pull between the shoulders.

An "unfolded" embodiment of the first embodiment of the device according to the invention is illustrated in fig. 11 with the collar 1, the shoulder straps 2 and the connecting band 3. When donning the device the user wraps the collar 1 tightly around one leg and slings and closes the straps 2 around the shoulders. The collar and/or the shoulder straps may be provided with one or more closing mechanisms, such as buckles or fabric hook-and-loop fasteners, or a combination of closing mechanisms. From fig. 11 it is seen that this embodiment is symmetrical around the long axis of the band 3. Thereby the illustrated device can be applied to both sides of the hip, i.e. there is no particular left side version or right side version. This helps to reduce the manufacturing costs. However, as indicated above in the description of the different embodiments there are various device features that may be different providing better comfort and/or better hip joint support. But these features possibly necessitate specific left and right side versions of the device. This is for example the case if one of the upper shoulder strap attachment sites is positioned further down the band as illustrated in fig. 10b.

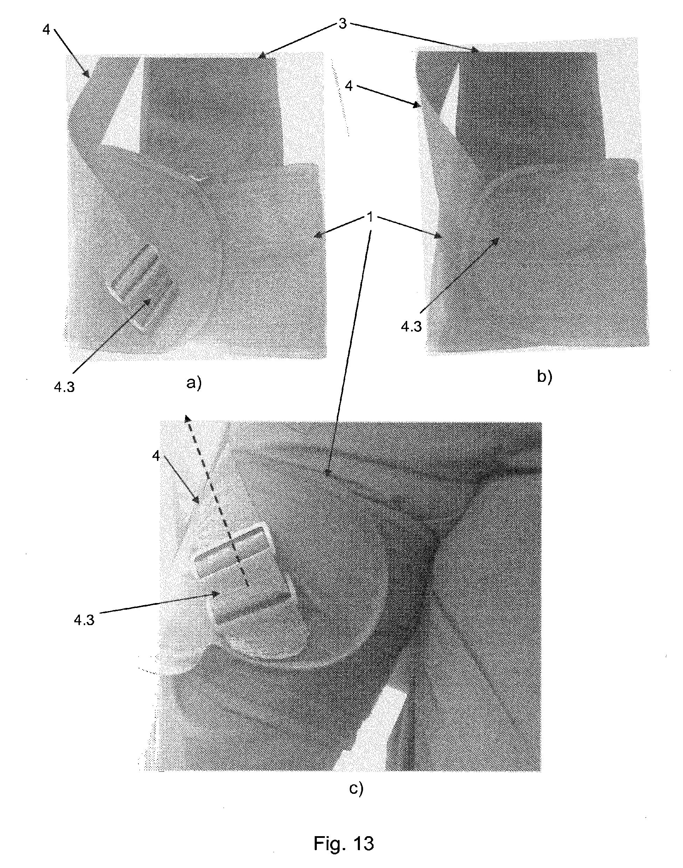

Fig. 12 show photos of a sixth and currently preferred embodiment of the invention. The device is worn by a tall man in fig. 12a (shown from the back) and fig. 12b (shown from the side and back) whereas the same device is worn by a less tall woman in fig. 12c. The device is shown partly in fig. 12d where it is lying on a floor. As seen from the figures this sixth embodiment is comparable to the first embodiment with the further addition of a fourth member 4 that is added to provide additional support to the hip joint, in particular to prevent adduction, i.e. crossing the legs. In this case where the device is attached to the right leg of the user, the fourth member will help prevent the user to adduct in the right side of the hip joint, i.e. preventing that the right leg crosses the left leg. This particular movement may pose a risk after hip surgery. The fourth member 4 may also help to prevent medial rotation of the right leg. In the embodiment in fig. 12 the fourth member 4 is attached to the upper part of the third member 3 at point 4.1, i.e. substantially the same place 2.1 as the second member 2 is attached to the third member 3. From the attachment point 4.1 the fourth member 4 extends down along the third member 3, almost diagonally along the back of the user, and a further attachment point between the third member 3 and the fourth member 4 is provided at point 4.2 along the vertical lower right edge of the third member 3. This can be seen from fig. 12d. As seen from fig. 12a the fourth member 4 extends further round the outside of the right thigh of the user. The fourth member 4 is attached to the front part of the first member, i.e. at the front part of the thigh. This attachment is not visible in fig. 12 but can be seen in fig. 13.

For the device according to the present invention it is typically required that the third member is taut to provide the desired functionality of limiting certain hip joint movements. To account for different height and anatomical measures of different users the device is thereby preferably adjustable, typically by having adjustment means on the second member, e.g. in form of buckles, such that the length of the second member can be adjusted. When tightening the second member the third member can be made taut on a user. The photos in figs. 12a and 12c show the same device can be adjusted to fit users of different height. However, on the tall man in fig. 12a the upper part of the third member 3 ends at the middle of his back whereas the upper part of the third member 3 extends to between the shoulder blades on the short woman in fig. 12c. The photos show that the same device can be adjusted to fit users of different height. But it also shows that is might be advantageously to manufacture the device in different sizes, e.g. small, medium and large as known from clothing. It will typically be the length of the third member 3 that will vary between these different device sizes.

Fig. 13 show close-up photos of the first member 1 and the attachment 4.3 between the first member 1 and the fourth member 4 of the sixth embodiment of the device. In this case the first member 1 is a collar with a Velcro closing mechanism that can conform tightly to the thigh of a user and the fourth member 4 is a nylon strap which is soft and flexible and substantially inelastic. Fig. 13a and 13b show different configurations of the attachment 4.3 between the fourth member 4 and the collar 1. Fig. 13c show a close-up of the collar 1 when mounted on a user sitting in a chair. The fourth member 4 may be provided with a buckle at the attachment point 4.3 enabling adjustment of the length of the fourth member 4 to account for different heights of different users. In figs. 13a and 13c the attachment point 4.3 is provided at the outside flap of the collar 1 whereas in fig. 13b the attachment point 4.3 is provided at the flap of the collar which extends round the inside of the thigh of the user. The configuration in fig. 13b may provide better protection against hip adduction and medial rotation because the tensional pull in the fourth member 4 has a direction indicated by the dotted arrow in fig. 13c, i.e. if the fourth member 4 is attached to the outside flap of the collar 1 as in figs. 13a and 13c the tensional pull from the fourth member 4 will tend to "open" the collar 1, whereas tensional pull in the fourth member 4 attached to the inside flap of the collar 1 as in fig. 13b will be more stable.

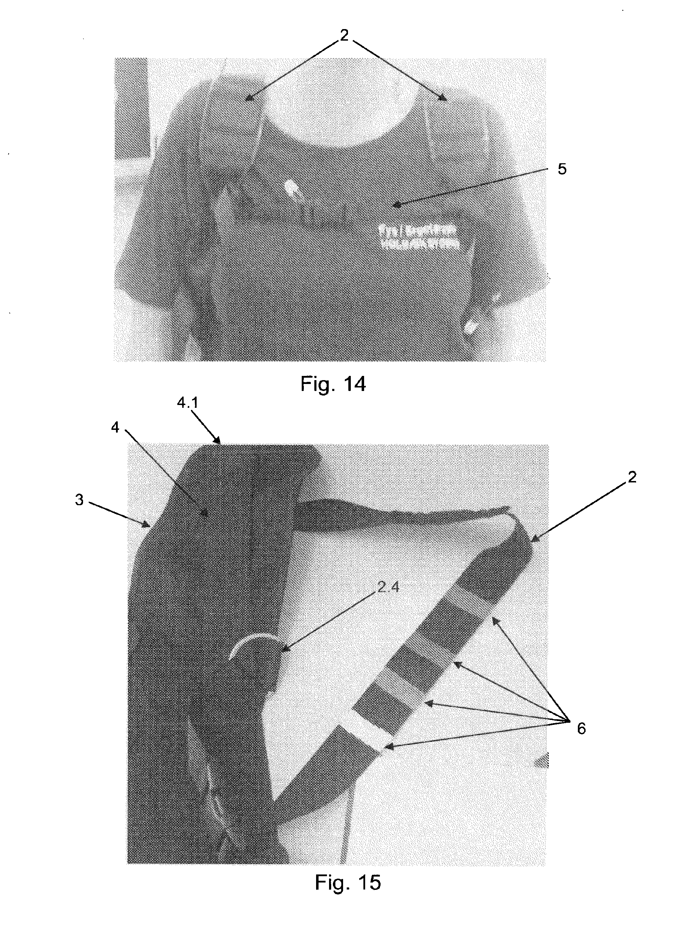

Fig. 14 shows a stabilising strap 5 that may be mounted on the second member 2, in this case shoulder straps 2. This principle is also known from rucksacks.

Fig. 15 shows a close-up photo of the second member 2 of one embodiment of the device. The second member 2 will typically be one or two shoulder straps and preferably the adjustment of the device to the correct tautness is provided by adjusting the second member, e.g. by means of buckles as known from backpacks and rucksacks. In this case the second member is shoulder straps and the photo in fig. 15 shows markings 6 provided on the strap 2. These markings are provided with different colours to assist a user of the device in adjusting the device to the correct tautness when adjusting the length of the shoulder straps. I.e. once the user knows the configuration of the device that suits his individual measures the markings 6 may help him to adjust the device to the same configuration each time the device is mounted.

## Claims

1. A device for limiting movement in a human hip joint, said device comprising:
- at least a first member (1) adapted to conform to a leg,
- a second member (2) adapted to engage at least one shoulder, and
- a third member (3) connecting said at least first member (1) and said second member (2),
the device **characterized in that** the third member (3) is a soft and flexible band adapted to
- extend from said leg across one buttock to the back, and configured to limit flexion and adduction in only the hip joint joining said leg,
or
- extend from said leg across the groin to the chest, and configured to limit extension and abduction in only the hip joint joining said leg,
by anchoring the tensile load of said movements in the shoulder(s).

2. The device according to any of the preceding claims, wherein the first member (1) is a collar, preferably adapted to conform to the thigh of the leg.

3. The device according to any of the preceding claims, wherein the second member (2) is one or more shoulder straps and/or wherein the second member (2) is provided with visible distinguishable markings.

4. The device according to any of the preceding claims, comprising only one shoulder strap or comprising two shoulder straps, one for each shoulder.

5. The device according to any of the preceding claims, wherein the third member (3) is substantially flat and wide and elongated.

6. The device according to any of the preceding claims, wherein the third member (3) is substantially inelastic and/or wherein the third member (3) is provided with a narrow end adapted to conform to a buttock and a leg of a user.

7. The device according to any of the preceding claims, further comprising a fourth member (4) attached to the first member (1) and the upper part of the third member (3).

8. The device according to claim 7, wherein the fourth member (4) is substantially soft and/or flexible and adapted to limit one or more specific movements of said leg by anchoring the tensile load of said movements in the shoulder(s).

9. The device according to any of claims 7 to 8, wherein the fourth member (4) is adapted to extend from the back to the front side of the thigh of a user.

10. The device according to any of claims 7 to 9, wherein the fourth member (4) is a strap and/or wherein the fourth member (4) is substantially inelastic.

11. The device according to any of the preceding claims, wherein the width of the third member (3) is between 5 and 26 cm, such as between 7 and 24 cm, such as between 10 and 22 cm, such as between 11 and 21 cm, such as between 12 and 20 cm, such as between 13 and 19 cm, such as between 14 and 18 cm, such as between 15 and 17 cm.

12. The device according to any of the preceding claims, wherein the length of the third member (3) is between 40 and 100 cm, such as between 50 and 90 cm, such as between 55 and 85 cm, such as between 60 and 80 cm, such as between 63 and 77 cm, such as between 65 and 75 cm, such as between 67 and 73 cm, such as between 69 and 71 cm.

13. The device according to any of the preceding claims, wherein the collar is adapted to conform around a thigh, preferably adapted to fit tightly around the lower part of the thigh and/or wherein the shape of the collar is substantially conical.

14. The device according to any of the preceding claims, wherein the device is primarily manufactured in textile selected from the group of fabric, cloth, woven fabric, twill, denim, chino, drill, gabardine, tweed, canvas and serge.

15. The device according to any of the preceding claims, wherein the first (1) and/or the third member (3) is primarily manufactured in textile selected from the group of fabric, cloth, woven fabric, twill, denim, chino, drill, gabardine, tweed, canvas and serge.

## Patentansprüche

1. Vorrichtung zur Einschränkung der Bewegung eines menschlichen Hüftgelenks, wobei diese Vorrichtung umfasst:
- mindestens ein erstes Element (1), das dazu eingerichtet ist, sich einem Bein anzupassen,
- ein zweites Element (2), das dazu eingerichtet ist, mit mindestens einer Schulter in Eingriff zu kommen, und
- ein drittes Element (3), welches das erste Element (1) und das zweite Element (2) verbindet,
wobei die Vorrichtung **dadurch gekennzeichnet ist, dass** das dritte Element (3) ein weiches und flexibles Band ist, das dazu eingerichtet ist,
- sich von dem Bein über eine Gesäßbacke zum Rücken erstrecken und dazu konfiguriert ist, die Flexion und Adduktion in nur dem Hüftgelenk dieses Beines zu beschränken,
oder
- sich von dem Bein über die Leiste zur Brust zu erstrecken und dazu konfiguriert ist, die Extension und Abduktion in nur dem Hüftgelenk dieses Beines zu beschränken,
indem die Zugbelastung dieser Bewegungen in der Schulter/den Schultern verankert wird.

2. Vorrichtung nach einem der vorhergehenden Ansprüche, wobei das erste Element (1) eine Manschette ist, die vorzugsweise dazu eingerichtet ist, sich dem Oberschenkel des Beines anzupassen.

3. Vorrichtung nach einem der vorhergehenden Ansprüche, wobei das zweite Element (2) aus einem oder mehreren Schultergurten besteht und/oder wobei das zweite Element (2) mit optisch unterscheidbaren Markierungen versehen ist.

4. Vorrichtung nach einem der vorhergehenden Ansprüche, umfassend nur einen Schultergurt oder umfassend zwei Schultergurte, jeweils einen für eine Schulter.

5. Vorrichtung nach einem der vorhergehenden Ansprüche, wobei das dritte Element (3) im Wesentlichen flach, breit und langgestreckt ist.

6. Vorrichtung nach einem der vorhergehenden Ansprüche, wobei das dritte Element (3) im Wesentlichen unelastisch ist und/oder wobei das dritte Element (3) mit einem schmalen Ende versehen ist, das zur Anpassung an eine Gesäßbacke und ein Bein eines Benutzers eingerichtet ist.

7. Vorrichtung nach einem der vorhergehenden Ansprüche, des Weiteren umfassend ein viertes Element (4), das an dem ersten Element (1) und dem oberen Teil des dritten Elements (3) befestigt ist.

8. Vorrichtung nach Anspruch 7, wobei das vierte Element (4) im Wesentlichen weich und/oder flexibel und dazu eingerichtet ist, eine oder mehrere spezifische Bewegungen des Beins zu beschränken, indem die Zugbelastung dieser Bewegungen in der Schulter/den Schultern verankert wird.

9. Vorrichtung nach einem der Ansprüche 7 bis 8, wobei das vierte Element (4) dazu eingerichtet ist, sich von der Rückseite zu der Vorderseite des Oberschenkels eines Benutzers zu erstrecken.

10. Vorrichtung nach einem der Ansprüche 7 bis 9, wobei das vierte Element (4) ein Gurt ist und/oder wobei das vierte Element (4) im Wesentlichen unelastisch ist.

11. Vorrichtung nach einem der vorhergehenden Ansprüche, wobei die Breite des dritten Elements (3) zwischen 5 und 26 cm beträgt, wie beispielsweise zwischen 7 und 24 cm, zwischen 10 und 22 cm, zwischen 11 und 21 cm, zwischen 12 und 20 cm, zwischen 13 und 19 cm, zwischen 14 und 18 cm, zwischen 15 und 17 cm.

12. Vorrichtung nach einem der vorhergehenden Ansprüche, wobei die Länge des dritten Elements (3) zwischen 40 und 100 cm beträgt, wie beispielsweise zwischen 50 und 90 cm, zwischen 55 und 85 cm, zwischen 60 und 80 cm, zwischen 63 und 77 cm, zwischen 65 und 75 cm, zwischen 67 und 73 cm, zwischen 69 und 71 cm.

13. Vorrichtung nach einem der vorhergehenden Ansprüche, wobei die Manschette dazu eingerichtet ist, um einen Oberschenkel herum zu passen und vorzugsweise dazu eingerichtet ist, um den unteren Teil des Oberschenkels zu passen und/oder wobei die Form der Manschette im Wesentlichen konisch ist.

14. Vorrichtung nach einem der vorhergehenden Ansprüche, wobei die Vorrichtung überwiegend aus Textilien hergestellt wird, die aus Gruppe aus Gewebe, Tuch, Vollgewebe, Twill, Jeans, Chino, Drillich, Gabardine, Tweed, Canvas und Serge gewählt werden.

15. Vorrichtung nach einem der vorhergehenden Ansprüche, wobei das erste (1) und/oder das dritte Element (3) überwiegend aus Textilien hergestellt werden, die aus Gruppe aus Gewebe, Tuch, Vollgewebe, Twill, Jeans, Chino, Drillich, Gabardine, Tweed, Canvas und Serge gewählt werden.

## Revendications

1. Dispositif pour limiter le mouvement dans une articulation de la hanche chez un être humain, ledit dispositif comprenant :
- au moins un premier élément (1) adapté pour se conformer à une jambe,
- un deuxième élément (2) adapté pour venir en prise avec au moins une épaule, et
- un troisième élément (3) reliant ledit ou lesdits premiers éléments (1) et ledit deuxième élément (2),
le dispositif étant **caractérisé en ce que** le troisième élément (3) est une bande souple et flexible adaptée pour
- s'étendre à partir de ladite jambe en passant par une fesse jusqu'au dos, et configuré pour limiter la flexion et l'adduction uniquement dans l'articulation de la hanche joignant ladite jambe,
ou
- s'étendre à partir de ladite jambe en passant par l'aine jusqu'au thorax, et configuré pour limiter l'extension et l'abduction uniquement dans l'articulation de la hanche joignant ladite jambe,
par ancrage de la charge de traction desdits mouvements dans l'épaule ou les épaules.

2. Dispositif selon l'une quelconque des revendications précédentes, dans lequel le premier élément (1) est une collerette, de préférence adaptée pour se conformer à la cuisse de la jambe.

3. Dispositif selon l'une quelconque des revendications précédentes, dans lequel le deuxième élément (2) est une ou plusieurs sangles d'épaule et/ou dans lequel le deuxième élément (2) est doté de repères visibles et différenciables.

4. Dispositif selon l'une quelconque des revendications précédentes, comprenant uniquement une sangle d'épaule ou deux sangles d'épaule, une pour chaque épaule.

5. Dispositif selon l'une quelconque des revendications précédentes, dans lequel le troisième élément (3) est sensiblement plat, large et allongé.

6. Dispositif selon l'une quelconque des revendications précédentes, dans lequel le troisième élément (3) est sensiblement inélastique et/ou dans lequel le troisième élément (3) est doté d'une extrémité étroite adaptée pour se conformer à une fesse et une jambe d'un utilisateur.

7. Dispositif selon l'une quelconque des revendications précédentes, comprenant en outre un quatrième élément (4) fixé au premier élément (1) et à la partie supérieure du troisième élément (3).

8. Dispositif selon la revendication 7, dans lequel le quatrième élément (4) est sensiblement souple et/ou flexible et adapté pour limiter un ou plusieurs mouvements spécifiques de ladite jambe par ancrage de la charge de traction desdits mouvements dans l'épaule ou les épaules.

9. Dispositif selon l'une quelconque des revendications 7 à 8, dans lequel le quatrième élément (4) est adapté pour s'étendre à partir du côté arrière jusqu'au côté avant de la cuisse d'un utilisateur.

10. Dispositif selon l'une quelconque des revendications 7 à 9, dans lequel le quatrième élément (4) est une sangle et/ou dans lequel le quatrième élément (4) est sensiblement inélastique.

11. Dispositif selon l'une quelconque des revendications précédentes, dans lequel la largueur du troisième élément (3) est entre 5 et 26 cm, par exemple entre 7 et 24 cm, par exemple entre 10 et 22 cm, par exemple entre 11 et 21 cm, par exemple entre 12 et 20 cm, par exemple entre 13 et 19 cm, par exemple entre 14 et 18cm, par exemple entre 15 et 17 cm.

12. Dispositif selon l'une quelconque des revendications précédentes, dans lequel la longueur du troisième élément (3) est entre 40 et 100 cm, par exemple entre 50 et 90 cm, par exemple entre 55 et 85 cm, par exemple entre 60 et 80 cm, par exemple entre 63 et 77 cm, par exemple entre 65 et 75 cm, par exemple entre 67 et 73 cm, par exemple entre 69 et 71 cm.

13. Dispositif selon l'une quelconque des revendications précédentes, dans lequel la collerette est adaptée pour se conformer autour d'une cuisse, de préférence adaptée pour s'ajuster étroitement autour de la partie inférieure de la cuisse et/ou dans lequel la forme de la collerette est sensiblement conique.

14. Dispositif selon l'une quelconque des revendications précédentes, dans lequel le dispositif est principalement fabriqué en un textile sélectionné dans le groupe du tissu, de la toile, du tissu tissé, du twill, du denim, du coutil, du drill, de la gabardine, du tweed, de la toile à canevas et du sergé.

15. Dispositif selon l'une quelconque des revendications précédentes, dans lequel le premier (1) et/ou le troisième (3) élément sont principalement fabriqués en un textile du groupe du tissu, de la toile, du tissu tissé, du twill, du denim, du coutil, du drill, de la gabardine, du tweed, de la toile à canevas et du sergé.
